# EUROPEAN PATENT APPLICATION

(11) **EP 4 846 855 A1**
(43) Date of publication of application: **02.12.2026**
(21) Application number: 25745514.7
(22) Date of filing: 24.01.2025
(51) Int. Cl.: A23J 3/14, A23J 3/00, A23L 11/00, A23L 25/00, C12N 9/54, C12N 9/56, C12P 21/00

(54) **AGENT FOR INHIBITING CHANGE IN TASTE OF VEGETABLE-PROTEIN-CONTAINING LIQUID COMPOSITION**

(30) Priority: 25.01.2024 JP 2024009750
(71) Applicant: Amano Enzyme Inc., Nagoya-shi Aichi 460-8630 (JP); Amano Enzyme U.S.A. Co., Ltd., Elgin, Illinois 60124 (US)
(72) Inventor: XU, Liangji, Elgin, Illinois 60124 (US); BROCHES, Nicholas, Elgin, Illinois 60124 (US); HASHIMURA, Dai, Elgin, Illinois 60124 (US)
(74) Representative: Vossius & Partner Patentanwälte Rechtsanwälte mbB
(86) International application number: PCT/JP2025/002337
(87) International publication number: WO 2025/159208

(57) **Abstract**

The purpose of the present invention is to provide a processing technique with which it is possible to suppress any change in taste while improving the solubility of a vegetable-protein-containing liquid composition (i.e., further increasing the amount of protein dissolved in water over that when the liquid composition is solubilized only by a protein deamidase). A vegetable-protein-containing liquid composition is treated with a protein deamidase and a bacteria-derived protease, whereby any change in taste resulting from solubilization of the liquid composition can be suppressed.

## Description

### Technical Field

The present invention relates to a processing technique for a plant-protein-containing liquid composition with which a change in taste caused by a solubilizing treatment of the liquid composition can be suppressed.

### Background Art

For various reasons such as a recent health boom, countermeasures against allergy problems, religious reasons, and increased voluntary restraint of going outdoors accompanying the spread of infectious diseases and the like, plant protein beverages (plant milk), which are rich in nutrients and can be stored for a long period of time, have become popular as an alternative to animal milk.

Meanwhile, plant proteins are generally inferior in properties such as solubility to proteins contained in animal milk, and thus use of plant proteins is inevitably limited. Therefore, plant milk cannot sufficiently substitute for animal milk, and use or application of plant milk has not been sufficiently achieved.

As a method for improving the solubility of plant protein, deamidation, that is, hydrolysis of an amide group in a side chain of a glutamine residue or an asparagine residue in a protein is known as an effective method. For example, PTL 1 discloses that aggregation of plant milk added to a high-temperature liquid food or beverage can be suppressed by treating the plant milk with a protein deamidase.

### Citation List

### Patent Literature

PTL 1: PCT International Publication No. 2020/171106

### Summary of Invention

### Technical Problem

In order to allow plant protein beverages to penetrate more widely and deeply into the general market, further improvement of processing techniques is desired. As a property to be obtained by processing, the solubility of protein is still regarded as important. As a means for improving the solubility of plant protein, the use of a protein deamidase is known, but the solubilizing effect thereof is limited. As a further solubilizing means, it is conceivable to combine a protease. However, since the protease has a point of action different from that of the protein deamidase, the protease may undesirably change the component composition of the plant protein beverage, leading to an undesired change in taste.

Therefore, an object of the present invention is to provide a processing technique with which a change in taste can be suppressed while improving the solubility of a plant-protein-containing liquid composition treated with a protein deamidase (i.e., increasing the amount of protein dissolved in water as compared with the case of solubilization only with a protein deamidase).

### Solution to Problem

The present inventors have found that by treating a plant-protein-containing liquid composition with a protein deamidase in combination with a bacteria-derived protease such as a protease derived from Bacillus licheniformis or a protease derived from Bacillus amyloliquefaciens, a change in taste can be suppressed with respect to taste in the case of solubilization only with a protein deamidase while improving the solubility as compared with the case of solubilization only with a protein deamidase. The present inventors have conducted further studies based on the findings, leading to the completion of the present invention.

That is, the present invention provides inventions of the following aspects.
Item 1. A taste change suppressing method of suppressing a change in taste while improving solubility of a plant-protein-containing liquid composition treated with a protein deamidase, the taste change suppressing method including:
   a step of treating the plant-protein-containing liquid composition with the protein deamidase and a bacteria-derived protease.
Item 2. The taste change suppressing method according to item 1, in which the bacteria-derived protease is selected from the group consisting of proteases derived from Bacillus licheniformis, Bacillus amyloliquefaciens, and corresponding genus Geobacillus.
Item 3. The taste change suppressing method according to item 1 or 2, in which the plant protein is selected from the group consisting of pea protein, soybean protein, and almond protein.
Item 4. The taste change suppressing method according to any one of items 1 to 3, in which a used amount of the bacteria-derived protease per 1 g of the plant protein is 13 to 250 U.
Item 5. The taste change suppressing method according to any one of items 1 to 4, in which a used amount of the protein deamidase per 1 g of the plant protein is 4 U or more.
Item 6. The taste change suppressing method according to any one of items 1 to 5, in which a used amount of the bacteria-derived protease per 1 U of the protein deamidase is 1 to 45 U.
Item 7. A taste change inhibitor containing a bacteria-derived protease and used for suppressing a change in taste while improving solubility of a plant-protein-containing liquid composition treated with a protein deamidase.
Item 8. The taste change inhibitor according to item 7, in which the bacteria-derived protease is selected from the group consisting of proteases derived from Bacillus licheniformis, Bacillus amyloliquefaciens, and corresponding genus Geobacillus.
Item 9. The taste change inhibitor according to item 7 or 8, in which the plant protein is selected from the group consisting of pea protein, soybean protein, and almond protein.
Item 10. The taste change inhibitor according to any one of items 7 to 9, which is further used for improving foamability of a plant-protein-containing liquid composition treated with a protein deamidase.
Item 11. The taste change inhibitor according to any one of items 7 to 10, which is further used for improving dispersion stability of a plant-protein-containing liquid composition treated with a protein deamidase.

### Advantageous Effects of Invention

According to the present invention, there is provided a processing technique with which it is possible to suppress a change in taste while improving the solubility of a plant-protein-containing liquid composition treated with a protein deamidase. Description of Embodiments

### 1. Taste change suppressing method

A taste change suppressing method of the present invention is a method of suppressing a change in taste with respect to taste in the case of solubilization only with a protein deamidase while improving the solubility of a plant-protein-containing liquid composition treated with a protein deamidase (i.e., increasing the amount of protein dissolved in water as compared with the case of solubilization only with a protein deamidase). Specifically, the taste change suppressing method of the present invention is a method of suppressing a change in taste while improving solubility of a plant-protein-containing liquid composition treated with a protein deamidase, the method including: a step of treating the plant-protein-containing liquid composition with the protein deamidase and a bacteria-derived protease. Preferably, the taste change suppressing method of the present invention further improves the foamability and/or dispersion stability of the plant-protein-containing liquid composition treated with a protein deamidase. Hereinafter, the taste change suppressing method of the present invention will be described in detail.

### 1-1. Plant-protein-containing liquid composition

The plant-protein-containing liquid composition used in the present invention is not particularly limited as long as it is liquid in which plant protein is dissolved and/or dispersed in water. Specific examples of the plant-protein-containing liquid composition include (i) a liquid obtained by dispersing dry powder (an aspect of a plant protein material) of a plant as an origin of plant protein (typically, a food product material) in water; (ii) a liquid obtained by dispersing dry powder (an aspect of a plant protein material) of a material, which is obtained by, for example, removing at least any of components other than the protein of a plant as an origin of plant protein (typically, a food product material) to increase the content of the protein, in water; (iii) a liquid obtained by crushing and dispersing a plant as an origin of plant protein (typically, a food product material, an aspect of a plant protein material) in water, and removing an insoluble matter derived from a skin or the like of the plant by any means such as centrifugation, filtration, filtration bag, or sieve, as necessary; (iv) a liquid and the like obtained by, for example, removing at least any of components other than the plant protein from the liquids of the above (i) to (iii) to increase the concentration of the protein; and (v) a liquid obtained by mixing dry powder (an aspect of a plant protein material) prepared from the liquid of any one of the above (i) to (iv) with water. As a preferred example of the plant-protein-containing liquid composition, plant milk is mentioned.

Examples of the plant protein include pea protein, soybean protein, and almond protein. These plant proteins may be used singly or in combination of a plurality of kinds thereof.

Among the origin plants of the plant protein described above, pea and soybean are preferable from the viewpoint of still further enhancing the effect of improving the solubility of the plant-protein-containing liquid composition treated with a protein deamidase.

Among the origin plants of the plant protein described above, almond is preferable from the viewpoint of still further enhancing the effect of improving the foamability of the plant-protein-containing liquid composition treated with a protein deamidase.

Among the origin plants of the plant protein described above, pea and soybean are preferable from the viewpoint of still further enhancing the effect of improving the dispersion stability of the plant-protein-containing liquid composition treated with a protein deamidase.

The content of the plant protein contained in the plant protein material is not particularly limited, and is, for example, 0.01 wt% or more, 0.05 wt% or more, 0.1 wt% or more, or 1 wt% or more, preferably 5 wt% or more, 10 wt% or more, or 20 wt% or more, and more preferably 30 wt% or more, or 35 wt% or more. The upper limit of the content range is not particularly limited, and is, for example, 95 wt% or less, preferably 90 wt% or less, and more preferably 82 wt% or less. The specific range of the content of the plant protein contained in the plant protein material is, for example, 0.01 to 95 wt%, 0.05 to 95 wt%, 0.1 to 95 wt%, or 1 to 90 wt%, preferably 5 to 90 wt%, 10 to 90 wt%, or 20 to 82 wt%, and more preferably 30 to 82 wt%, or 35 to 82 wt%. The lower limit value of the specific range may be 40 wt%, 50 wt%, 60 wt%, 70 wt%, or 75 wt%, and the upper limit value of the specific range may be 60 wt%, 50 wt%, 45 wt%, or 40 wt%.

More specific examples of the content of the plant protein contained in the plant protein material include the following.
· Content of the pea protein contained in the pea protein material: preferably 20 wt% or more, more preferably 40 wt% or more, further preferably 60 wt% or more, and still more preferably 70 wt% or more, or 75 wt% or more. Preferably 95 wt% or less, more preferably 90 wt% or less, and further preferably 82 wt% or less. Specifically, preferably 20 to 95 wt%, more preferably 40 to 90 wt%, further preferably 60 to 90 wt%, and still more preferably 70 to 82 wt%, or 75 to 82 wt%.
· Content of the soybean protein contained in the soybean protein material: preferably 10 wt% or more, more preferably 20 wt% or more, further preferably 30 wt% or more, and still more preferably 35 wt% or more. Preferably 60 wt% or less, more preferably 50 wt% or less, and further preferably 45 wt% or less, or 40 wt% or less. Specifically, preferably 10 to 60 wt%, more preferably 20 to 50 wt%, further preferably 30 to 45 wt%, and still more preferably 35 to 40 wt%.
· Content of the almond protein contained in the almond protein material: preferably 10 wt% or more, more preferably 20 wt% or more, further preferably 30 wt% or more, or 35 wt% or more, and still more preferably 40 wt% or more. Preferably 60 wt% or less, more preferably 50 wt% or less, and further preferably 45 wt% or less. Specifically, preferably 10 to 60 wt%, more preferably 20 to 50 wt%, further preferably 30 to 50 wt%, or 35 to 45 wt%, and still more preferably 40 to 45 wt%.

The content (in terms of dry weight) of the plant protein material in the plant-protein-containing liquid composition is not particularly limited, and is, for example, 0.1 to 50 wt%, 0.5 to 40 wt%, or 1 to 30 wt%, preferably 1.5 to 25 wt%, 1.5 to 15 wt%, or 2 to 10 wt%, and more preferably 2.5 to 7 wt%, 3 to 6.5 wt%, 3 to 5 wt%, 3 to 4 wt%, 3 to 7 wt%, 4 to 7 wt%, 5 to 7 wt%, or 5.5 to 7 wt%.

The content (in terms of dry weight) of the plant protein material in the plant-protein-containing liquid composition is more specifically as follows.
· Content (in terms of dry weight) of the pea protein material: Preferably 1.5 to 25 wt%, more preferably 2 to 7 wt%, further preferably 2.5 to 6.5 wt%, and still more preferably 3 to 5 wt%, or 3 to 4 wt%.
· Content (in terms of dry weight) of the soybean protein material: Preferably 1.5 to 25 wt%, more preferably 2 to 7 wt%, further preferably 2.5 to 6.5 wt%, and still more preferably 3 to 5 wt%, or 3 to 4 wt%.
· Content (in terms of dry weight) of the almond protein material: Preferably 3 to 25 wt%, more preferably 4 to 15 wt%, further preferably 5 to 10 wt%, and still more preferably 5.5 to 7 wt%.

The content of the plant protein in the plant-protein-containing liquid composition is not particularly limited, and is, for example, 0.001 to 50 wt%, 0.005 to 40 wt%, 0.01 to 20 wt%, 0.05 to 15 wt%, 0.1 to 10 wt%, or 0.3 to 7 wt%, preferably 0.6 to 4 wt%, more preferably 0.8 to 3 wt%, and further preferably 1 to 2.7 wt%, 1 to 2 wt%, 1 to 1.5 wt%, 1.5 to 3 wt%, or 2 to 3 wt%.
· Content of the pea protein: Preferably 0.6 to 4 wt%, more preferably 1 to 3 wt%, further preferably 1.5 to 3 wt%, and still more preferably 2 to 3 wt%.
· Content of the soybean protein: Preferably 0.6 to 4 wt%, more preferably 0.8 to 3 wt%, further preferably 1 to 2.7 wt%, and still more preferably 1 to 2 wt%, or 1 to 1.5 wt%.
· Content of the almond protein: Preferably 0.6 to 4 wt%, more preferably 1 to 3 wt%, further preferably 1.5 to 3 wt%, and still more preferably 2 to 3 wt%.

### 1-2. Protein deamidase

The type, origin, and the like of the protein deamidase that treats the plant-protein-containing liquid composition are not particularly limited as long as the protein deamidase is an enzyme that exhibits an action of decomposing an amide group-containing side chain of a protein without cleaving peptide bonds and without crosslinking the protein.

Examples of the protein deamidase include an enzyme that deamidates a glutamine residue in a protein and converts it into glutamic acid (for example, protein glutaminase), and an enzyme that deamidates an asparagine residue in a protein and converts it into aspartic acid (for example, protein asparaginase).

More specific examples of the protein deamidase include a protein deamidase derived from the genus Chryseobacterium, Flavobacterium, Empedobacter, Sphingobacterium, Aureobacterium, Myroides, Luteimicrobium, Agromyces, Microbacterium, or Leifsonia. These protein deamidases are known, and for example, JP 2000-50887 A, JP 2001-218590 A, WO 2006/075772 A1, WO 2015/133590, and the like can be referred to. These protein deamidases may be used singly or in combination of a plurality of kinds thereof.

Among these protein deamidases, from the viewpoint of still further enhancing the solubility improving effect and/or the taste change suppressing effect, or foamability and/or dispersion stability in addition to these effects, a protein deamidase derived from the genus Chryseobacterium is preferable, a protein glutaminase derived from the genus Chryseobacterium is more preferable, a protein glutaminase derived from Chryseobacterium proteolyticum species is further preferable, and a protein glutaminase derived from the Chryseobacterium proteolyticum 9670 strain is still more preferable.

The protein deamidase can be prepared from a culture broth of a microorganism from which the protein deamidase is derived. Specific examples of the preparation method include a method of recovering a protein deamidase from a culture broth or a bacterial cell of the above-mentioned microorganism. For example, in the case of using a microorganism that secretes a protein deamidase, an enzyme can be separated and/or purified after recovering bacterial cells from the culture broth in advance by filtration, a centrifugal treatment, or the like, as necessary. In the case of using microorganism that does not secrete a protein deamidase, an enzyme can be separated and/or purified after recovering bacterial cells from the culture broth in advance as necessary and then disrupting the bacterial cells by a pressurization treatment, an ultrasonic treatment, or the like to expose an enzyme. As an enzyme separation and/or purification method, a known protein separation and/or purification method can be used without particular limitation, and examples thereof include a centrifugal separation method, a UF concentration method, a salting-out method, and various chromatography methods using an ion-exchange resin or the like. The separated and/or purified enzyme can be powdered by a drying method such as freeze-drying or reduced-pressure drying, and can also be powdered using an appropriate excipient and/or drying aid in the drying method. The separated and/or purified enzyme can also be liquefied by adding an appropriate additive and performing filtration sterilization.

The used amount of the protein deamidase is not particularly limited, and the used amount per 1 g of the plant protein is, for example, 4 U or more. From the viewpoint of still further enhancing the solubility improving effect and/or the taste change suppressing effect, or foamability and/or dispersion stability in addition to these effects, the used amount of the protein deamidase per 1 g of the plant protein is preferably 4.5 U or more, and more preferably 4.8 U or more. The upper limit of the used amount range of the protein deamidase per 1 g of the plant protein is not particularly limited, and is, for example, 20 U or less, 15 U or less, 11 U or less, 10 U or less, or 5.5 U or less. The specific range of the used amount of the protein deamidase per 1 g of the plant protein is, for example, 4 to 20 U, preferably 4.5 to 15 U, 4.5 to 11 U, or 4.5 to 10 U, and more preferably 4.8 to 5.5 U.

The used amount of the protein deamidase per 1 g of the plant protein material is, for example, 0.01 U or more. From the viewpoint of still further enhancing the solubility improving effect and/or the taste change suppressing effect, or foamability and/or dispersion stability in addition to these effects, the used amount of the protein deamidase per 1 g of the plant protein material is preferably 1 U or more, more preferably 1.5 U or more, and further preferably 1.8 U or more. The upper limit of the used amount range of the protein deamidase per 1 g of the plant protein material is not particularly limited, and is, for example, 15 U or less, 8 U or less, or 5 U or less. A specific range of the used amount of the protein deamidase per 1 g of the plant protein material is, for example, 0.01 to 15 U, preferably 1 to 15 U, more preferably 1.5 to 8 U, and further preferably 1.8 to 5 U.

For the activity of the protein deamidase, benzyloxycarbonyl-L-glutaminylglycine (Z-Gln-Gly) is used as a substrate, and the amount of enzyme that liberates 1 µmol of ammonia per minute is defined as 1 unit (1 U).

### 1-3. Bacteria-derived protease

The bacteria-derived protease is not particularly limited as long as it is an enzyme derived from bacteria that hydrolyzes a peptide bond of a protein. The bacteria-derived protease can suppress a change in taste while further enhancing the solubility improving effect by the protein deamidase.

Examples of the bacteria-derived protease include proteases derived from Bacillus licheniformis, Bacillus amyloliquefaciens, and corresponding genus Geobacillus (that is, Geobacillus licheniformis, Geobacillus amyloliquefaciens). Hereinafter, the genus Bacillus and the genus Geobacillus are collectively referred to as "the genus (Geo)Bacillus".

As the bacteria-derived protease, these proteases may be used singly or in combination of two or more kinds thereof. Among these bacteria-derived proteases, from the viewpoint of still further enhancing the solubility improving effect and/or the taste change suppressing effect, or foamability and/or dispersion stability in addition to these effects, a protease derived from (Geo)bacillus amyloliquefaciens is preferable, and a protease derived from Bacillus amyloliquefaciens is more preferable.

The bacteria-derived protease can be prepared by a known method. As an example, when the protease derived from the genus (Geo)Bacillus is prepared, the protease can be easily prepared by a method of culturing a bacterium belonging to the genus (Geo)Bacillus and separating the protease using a known means, a method using a genetic recombination technique, or the like. As the bacteria-derived protease, a commercially available product may be used.

The used amount of the bacteria-derived protease is not particularly limited, but the used amount per 1 g of the plant protein is, for example, 13 to 250 U.

More specifically, as the used amount of the protease derived from (Geo)bacillus licheniformis per 1 g of the plant protein, the following used amounts are preferable from the viewpoint of still further enhancing the solubility improving effect and/or the taste change suppressing effect, or foamability and/or dispersion stability in addition to these effects.
· Used amount of the protease derived from (Geo)bacillus licheniformis per 1 g of the pea protein: Preferably 15 to 200 U, more preferably 30 to 200 U, and further preferably 45 to 200 U, 45 to 150 U, 45 to 120 U, 48 to 110 U, 48 to 80 U, 48 to 60 U, or 48 to 55 U.
· Used amount of the protease derived from (Geo)bacillus licheniformis per 1 g of the soybean protein: Preferably 15 to 250 U, more preferably 30 to 250 U, further preferably 90 to 250 U, or 100 to 250 U, and still more preferably 160 to 250 U, 180 to 250 U, 190 to 250 U, or 190 to 230 U.
· Used amount of the protease derived from (Geo)bacillus licheniformis per 1 g of the almond protein: Preferably 13 to 150 U, more preferably 13 to 120 U, and further preferably 15 to 110 U (the lower limit value of the range may be 30 U, 50 U, 70 U, or 90 U, and the upper limit value of the range may be 100 U, 80 U, 60 U, 40 U, or 20 U).

More specifically, as the used amount of the protease derived from (Geo)bacillus amyloliquefaciens per 1 g of the plant protein, the following used amounts are preferable from the viewpoint of still further enhancing the solubility improving effect and/or the taste change suppressing effect, or foamability and/or dispersion stability in addition to these effects.
· Used amount of the protease derived from (Geo)bacillus amyloliquefaciens per 1 g of the pea protein: Preferably 13 to 250 U, or 15 to 250 U, more preferably 35 to 230 U, or 35 to 200 U, further preferably 45 to 140 U, 48 to 140 U, or 50 to 140 U, and still more preferably 80 to 140 U, or 90 to 120 U.
· Used amount of the protease derived from (Geo)bacillus amyloliquefaciens per 1 g of the soybean protein: Preferably 15 to 250 U, more preferably 30 to 250 U, or 50 to 250 U, further preferably 70 to 230 U, or 80 to 200 U, and still more preferably 90 to 150 U.
· Used amount of the protease derived from (Geo)bacillus amyloliquefaciens per 1 g of the almond protein: Preferably 13 to 150 U, more preferably 13 to 120 U, and further preferably 15 to 110 U (the lower limit value of the range may be 30 U, 50 U, 70 U, or 90 U, and the upper limit value of the range may be 100 U, 80 U, 60 U, 40 U, or 20 U).

The used amount of the bacteria-derived protease per 1 g of the plant protein material is, for example, 5 to 200 U.

More specifically, as the used amount of the protease derived from (Geo)bacillus licheniformis per 1 g of the plant protein material, the following used amounts are preferable from the viewpoint of still further enhancing the solubility improving effect and/or the taste change suppressing effect, or foamability and/or dispersion stability in addition to these effects.
· Used amount of the protease derived from (Geo)bacillus licheniformis per 1 g of the pea protein material: Preferably 12 to 160 U, more preferably 24 to 160 U, and further preferably 36 to 160 U, 36 to 120 U, 36 to 96 U, 38 to 88 U, 38 to 64 U, 38 to 48 U, or 38 to 44 U.
· Used amount of the protease derived from (Geo)bacillus licheniformis per 1 g of the soybean protein material: Preferably 5 to 100 U, more preferably 10 to 100 U, further preferably 35 to 100 U, or 38 to 100 U, and still more preferably 60 to 100 U, 70 to 100 U, 75 to 100 U, or 75 to 90 U.
· Used amount of the protease derived from (Geo)bacillus licheniformis per 1 g of the almond protein material: Preferably 5 to 60 U, more preferably 5 to 48 U, and further preferably 6 to 45 U (the lower limit value of the range may be 12 U, 20 U, 30 U, or 35 U, and the upper limit value of the range may be 40 U, 32 U, 24 U, 16 U, or 8 U).

More specifically, as the used amount of the protease derived from (Geo)bacillus amyloliquefaciens per 1 g of the plant protein material, the following used amounts are preferable from the viewpoint of still further enhancing the solubility improving effect and/or the taste change suppressing effect, or foamability and/or dispersion stability in addition to these effects.
· Used amount of the protease derived from (Geo)bacillus amyloliquefaciens per 1 g of the pea protein material: Preferably 10 to 200 U, or 12 to 200 U, more preferably 28 to 185 U, or 28 to 160 U, further preferably 36 to 110 U, 38 to 110 U, or 40 to 110 U, and still more preferably 64 to 110 U, or 72 to 95 U.
· Used amount of the protease derived from (Geo)bacillus amyloliquefaciens per 1 g of the soybean protein material: Preferably 5 to 100 U, more preferably 10 to 100 U, or 20 to 100 U, further preferably 27 to 90 U, or 30 to 80 U, and still more preferably 35 to 60 U.
· Used amount of the protease derived from (Geo)bacillus amyloliquefaciens per 1 g of the almond protein material: Preferably 5 to 60 U, more preferably 5 to 48 U, and further preferably 6 to 45 U (the lower limit value of the range may be 12 U, 20 U, 28 U, or 36 U, and the upper limit value of the range may be 40 U, 32 U, 24 U, 16 U, or 8 U).

The protease activity is measured using casein as a substrate by the Folin method. That is, the protease activity is determined in such a manner that an enzymatic reaction is performed using casein as a substrate by a conventional method, and the amount of enzyme that causes an increase in colored materials by Folin's reagent corresponding to 1 µg of tyrosine per minute is defined as 1 unit (1 U).

The used amount of the bacteria-derived protease per 1 U of the protein deamidase is, for example, 1 to 45 U.

More specifically, as the used amount of the protease derived from (Geo)bacillus licheniformis per 1 U of the protein deamidase, the following used amounts are preferable from the viewpoint of still further enhancing the solubility improving effect and/or the taste change suppressing effect, or foamability and/or dispersion stability in addition to these effects.
· Used amount of the protease derived from (Geo)bacillus licheniformis per 1 U of the protein deamidase in the case of using pea protein as a plant protein: Preferably 3 to 40 U, more preferably 6 to 40 U, and further preferably 9 to 40 U, 9 to 30 U, 9 to 25 U, 9.5 to 22 U, 9.5 to 16 U, 9.5 to 12 U, or 9.5 to 11 U.
· Used amount of the protease derived from (Geo)bacillus licheniformis per 1 U of the protein deamidase in the case of using soybean protein as a plant protein: Preferably 1.5 to 25 U, more preferably 2.8 to 25 U, further preferably 9 to 25 U, or 9.5 to 25 U, and still more preferably 16 to 25 U, 18 to 25 U, 19 to 25 U, or 19 to 23 U.
· Used amount of the protease derived from (Geo)bacillus licheniformis per 1 U of the protein deamidase in the case of using almond protein as a plant protein: Preferably 2.5 to 30 U, more preferably 2.5 to 24 U, and further preferably 3 to 22 U (the lower limit value of the range may be 6 U, 10 U, 14 U, or 18 U, and the upper limit value of the range may be 20 U, 16 U, 12 U, 8 U, or 4 U).

More specifically, as the used amount of the protease derived from (Geo)bacillus amyloliquefaciens per 1 U of the protein deamidase, the following used amounts are preferable from the viewpoint of still further enhancing the solubility improving effect and/or the taste change suppressing effect, or foamability and/or dispersion stability in addition to these effects.
· Used amount of the protease derived from (Geo)bacillus amyloliquefaciens per 1 U of the protein deamidase in the case of using pea protein as a plant protein: Preferably 2.5 to 45 U, or 5 to 45 U, more preferably 7 to 45 U, or 7 to 40 U, further preferably 9 to 28 U, 9.5 to 28 U, or 10 to 28 U, and still more preferably 16 to 28 U, or 18 to 24 U.
· Used amount of the protease derived from (Geo)bacillus amyloliquefaciens per 1 U of the protein deamidase in the case of using soybean protein as a plant protein: Preferably 1.5 to 25 U, more preferably 2.8 to 25 U, or 5 to 25 U, further preferably 7 to 23 U, or 8 to 20 U, and still more preferably 9 to 15 U.
· Used amount of the protease derived from (Geo)bacillus amyloliquefaciens per 1 U of the protein deamidase in the case of using almond protein as a plant protein: Preferably 2.5 to 30 U, more preferably 2.5 to 24 U, and further preferably 3 to 22 U (the lower limit value of the range may be 6 U, 10 U, 14 U, or 18 U, and the upper limit value of the range may be 20 U, 16 U, 12 U, 8 U, or 4 U).

### 1-4. Reaction conditions, etc.

In the step of treating a plant-protein-containing liquid composition with the protein deamidase and a bacteria-derived protease, the order of actions of the bacteria-derived protease and the protein deamidase is not particularly limited, and the enzymes may be sequentially made to act in any order, or both the enzymes may be simultaneously made to act.

The treatment temperature with the bacteria-derived protease and the protein deamidase is not particularly limited, and can be appropriately determined by those skilled in the art according to the optimum temperature of the enzyme to be used and/or the thermal properties of the plant-protein-containing liquid composition, and the like, but is, for example, 40 to 70°C, and preferably 48 to 62°C.

The enzyme treatment time of the plant-protein-containing liquid composition is not particularly limited, and may be appropriately determined according to the preparation scale of the composition, the timing of adding the enzymes, and the like, but is, for example, 30 minutes or longer and preferably 50 minutes or longer. The upper limit of the range of the enzyme treatment time is not particularly limited, and is, for example, 80 minutes or shorter, 70 minutes or shorter, or 60 minutes or shorter. The specific range of the enzyme treatment time is, for example, 30 to 80 minutes, or 30 to 70 minutes, and preferably 50 to 60 minutes.

The plant-protein-containing liquid composition after completion of the enzyme treatment is subjected to an enzyme inactivation step, then cooled, and subjected to a post-treatment such as filtration as necessary to obtain a processed plant-protein-containing liquid composition.

The obtained processed plant-protein-containing liquid composition is processed such that a change in taste is suppressed while the solubility is improved as compared with a case where the plant-protein-containing liquid composition is treated only with a protein deamidase. Furthermore, in a preferred aspect of the obtained processed plant-protein-containing liquid composition, the processed plant-protein-containing liquid composition is processed such that foamability and/or dispersion stability is also improved as compared with a case where the plant-protein-containing liquid composition is treated only with a protein deamidase.

Note that, the obtained processed plant-protein-containing liquid composition can also be prepared as a solid plant protein composition having improved protein solubility in water while suppressing a change in taste when being redispersed in water through a drying step, preferably as a solid plant protein composition having improved foamability and/or dispersion stability when being redispersed in water. The drying method is not particularly limited, and examples thereof include freeze drying, vacuum drying, and spray drying. Examples of the shape of the solid plant protein composition include powder, fine particles, and granules.

### 2. Taste change inhibitor for plant-protein-containing liquid composition

As described above, the bacteria-derived protease can suppress a change in taste of the plant-protein-containing liquid composition treated with a protein deamidase while improving the protein solubility in water. Therefore, the present invention also provides a taste change inhibitor containing a bacteria-derived protease and used for suppressing a change in taste while improving solubility of a plant-protein-containing liquid composition treated with a protein deamidase.

Specific aspects of use of the taste change inhibitor of the present invention include all aspects in which the taste change inhibitor and the protein deamidase are simultaneously used to treat the plant-protein-containing liquid composition, aspects in which the plant-protein-containing liquid composition is treated with the protein deamidase and then treated with the taste change inhibitor, and aspects in which the plant-protein-containing liquid composition is treated with the taste change inhibitor and then treated with the protein deamidase.

In a preferred aspect of the taste change inhibitor of the present invention, the taste change inhibitor is used for improving the foamability and/or dispersion stability of the plant-protein-containing liquid composition treated with a protein deamidase, in addition to being used for improving the solubility of the plant-protein-containing liquid composition treated with a protein deamidase and suppressing a change in taste.

In the above-described taste change inhibitor, the type and used amount of the bacteria-derived protease, a target plant-protein-containing liquid composition, the protein deamidase used in combination and used amount thereof, and the like are as shown in the above section "1. Taste change suppressing method".

### Examples

Hereinafter, the present invention will be specifically described by means of Examples; however, the present invention is not to be construed as being limited to the following Examples.

### [Materials and enzymes used]

**[Table 1A]**

| Plant protein material | Product name | Protein content | Manufacturer |
|---|---|---|---|
| Pea protein material | Puris 870 Pea Protein | 80 wt% | Puris |
| Soybean protein material | Organic Soy Flour | 38.5 wt% | Naturevibe Botanicals |
| Almond protein material | Blan Almond Protein Powder | 41 wt% | Blue Diamond Almonds |

**[Table 1B]**

| Enzyme | Origin | Manufacturer |
|---|---|---|
| Protease 1 | Bacteria (Bacillus licheniformis) | Amano Enzyme Inc. |
| Protease 2 | Bacteria (Bacillus amyloliquefaciens) | Amano Enzyme Inc. |
| Protein deamidase | Chryseobacterium proteolyticum | Amano Enzyme Inc. |

### [Enzyme activity measurement method]

### (1) Protease activity measurement method

After 5 mL of a 0.6% (w/v) casein solution (0.05 mol/L of sodium hydrogen phosphate, pH 8.0) was warmed at 37°C for 10 minutes, 1 mL of a sample solution containing a protease was added, and the mixture was immediately shaken. After this solution was left to stand at 37°C for 10 minutes, 5 mL of a trichloroacetic acid reagent (containing 1.8% (w/v) trichloroacetic acid, 1.8% (w/v) sodium acetate, and 0.33 mol/L acetic acid) was added, the mixture was shaken, and left to stand at 37°C for 30 minutes again, and the mixture was filtered. The first filtrate (3 mL) was removed, the next filtrate (2 mL) was measured, 5 mL of a 0.55 mol/L sodium carbonate reagent and 1 mL of Folin's reagent (1 → 3) were added, and the mixture was shaken well and left to stand at 37°C for 30 minutes. An absorbance AT of this solution (enzymatic reaction solution) at a wavelength of 660 nm was measured using water as a control.

Separately, a solution (blank) was prepared by performing the same operation as in the enzymatic reaction solution except that 1 mL of a sample solution containing a protease was measured, 5 mL of a trichloroacetic acid reagent (containing 1.8% (w/v) trichloroacetic acid, 1.8% (w/v) sodium acetate, and 0.33 mol/L acetic acid) was added, the mixture was shaken, 5 mL of a 0.6% (w/v) casein solution (0.05 mol/L sodium hydrogen phosphate, pH 8.0) was then added, the mixture was immediately shaken and left to stand at 37°C for 30 minutes, and an absorbance AB of this solution was measured.

The amount of enzyme that causes an increase in colored materials by Folin's reagent corresponding to 1 µg of tyrosine per minute was defined as 1 unit (1 U).

Each of 1 mL, 2 mL, 3 mL, and 4 mL of a 1 mg/mL tyrosine standard stock solution (0.2 mol/L of hydrochloric acid) was measured, and a 0.2 mol/L hydrochloric acid reagent was added thereto to make 100 mL. Each solution (2 mL) was measured, 5 mL of a 0.55 mol/L sodium carbonate reagent and 1 mL of Folin's reagent (1 → 3) were added, and the mixture was immediately shaken and left to stand at 37°C for 30 minutes. For these solutions, absorbances A1, A2, A3, and A4 at a wavelength of 660 nm were measured using, as a control, a solution obtained by measuring 2 mL of a 0.2 mol/L hydrochloric acid reagent and performing the same operation as described above. The absorbances A1, A2, A3, and A4 were plotted on the vertical axis and the amount (µg) of tyrosine in 2 mL of each solution was plotted on the horizontal axis to prepare a calibration curve, and the amount (µg) of tyrosine with respect to the absorbance difference of 1 was determined. [Mathematical Formula 1] $Protease activity \left(U/g, U/mL\right) = \left(AT-AB\right) \times F \times 11 / 2 \times 1 / 10 \times 1 / M$
AT: Absorbance of enzymatic reaction solution
AB: Absorbance of blank
F: Tyrosine amount (µg) when absorbance difference determined from tyrosine calibration curve is 1
11/2: Conversion coefficient to total liquid amount after stopping reaction
1/10: Conversion coefficient per minute of reaction time
M: Amount (g or mL) of sample in 1 mL of sample solution

### (2) Protein deamidase activity value measurement method

To 1 mL of a 0.2 M phosphate buffer (pH 6.5) containing 30 mM Z-Gln-Gly, 0.1 mL of a sample solution containing a protein deamidase was added, the mixture was left to stand at 37°C for 10 minutes, and then 1 mL of a 0.4 M TCA (trichloroacetic acid) solution was added to stop the reaction. As a blank, to 1 mL of a 0.2 M phosphate buffer (pH 6.5) containing 30 mM Z-Gln-Gly, 1 mL of a 0.4 M TCA solution was added, 0.1 mL of a sample solution containing a protein deamidase was further added, and the mixture was left to stand at 37°C for 10 minutes.

The amount of ammonia generated in the reaction solution was measured for the solution obtained above using Ammonia Test Wako (FUJIFILM Wako Pure Chemical Corporation). The ammonia concentration in the reaction solution was determined from a calibration curve representing the relationship between the ammonia concentration and the absorbance (630 nm) prepared using an ammonia standard solution (ammonium chloride).

The activity of the protein deamidase was calculated from the following formula with the amount of enzyme that produces 1 µmol of ammonia per minute being defined as 1 unit (1 U). In the formula, the reaction solution amount is 2.1, the enzyme solution amount is 0.1, and Df is a dilution factor of the enzyme solution. 17.03 is a molecular weight of ammonia. [Mathematical Formula 2] $\begin{matrix}Protein deamidase activity \left(U/mL\right) \\ = Ammonia concentration in reaction solution \left(mg/L\right) \times \left(1/17.03\right) \times \left(\begin{matrix}Reaction \\ solution amount / Enzyme solution amount\end{matrix}\right) \times \left(1/10\right) \times Df\end{matrix}$

### [Test Example]

### (1) Production of processed plant-protein-containing liquid composition

### (1-1) Production of processed pea protein-containing liquid composition

1.565 g of a pea protein material was added with 50 mL of water and suspended, and the mixture was pre-incubated at 50°C for 10 minutes under stirring. Protein glutaminase (protein deamidase) and various proteases were added in the amounts shown in Table 2A, and the mixture was treated at 50°C for 1 hour. Thereafter, the mixture was treated at 85°C for 15 minutes to inactivate the enzyme activity. After enzyme inactivation, the mixture was cooled to room temperature to obtain a processed pea protein-containing liquid composition.

### (1-2) Production of processed soybean protein-containing liquid composition

1.565 g of a soybean protein material was added with 50 mL of water and suspended, and the mixture was pre-incubated at 50°C for 10 minutes under stirring. Protein glutaminase and various proteases were added in the amounts shown in Table 3A, and the mixture was treated at 50°C for 1 hour. Thereafter, the mixture was treated at 85°C for 15 minutes to inactivate the enzyme activity. After enzyme inactivation, the mixture was cooled to room temperature to obtain a processed soybean protein-containing liquid composition.

### (1-3) Production of processed almond protein-containing liquid composition

3.05 g of an almond protein material was added with 50 mL of water and suspended, and the mixture was pre-incubated at 50°C for 10 minutes under stirring. Protein glutaminase and various proteases were added in the amounts shown in Table 4A, and the mixture was treated at 50°C for 1 hour. Thereafter, the mixture was treated at 85°C for 15 minutes to inactivate the enzyme activity. After enzyme inactivation, the mixture was cooled to room temperature to obtain a processed almond protein-containing liquid composition.

### (2) Evaluation of solubility improving effect

The processed plant-protein-containing liquid composition obtained in the above (1) was suspended by vortexing, and then centrifuged at 3,000 rpm for 20 minutes. The supernatant was collected so as not to take the cloudy upper layer, and the soluble protein was analyzed in an external institution. The analysis was performed using FP828 Series Combustion (Dumas method) manufactured by LECO Corporation. The soluble protein amount was calculated by the following calculation formula. In the following formula, 6.25 is a nitrogen-protein conversion coefficient. [Mathematical Formula 3] $Soluble protein amount = \left\{\left(\begin{matrix}Nitrogen amount in supernatant \times \\ 6.25\end{matrix}\right)/Supernatant weight\right\} \times 100$

When the soluble protein amount in the processed plant-protein-containing liquid composition obtained by using the protein deamidase alone without using a protease was taken as 1, the relative value of the soluble protein amount in the processed plant-protein-containing liquid composition of each of Examples and Comparative Examples was calculated. The results are shown in Tables 2A to 4A.

### (3) Evaluation of taste change suppressing property

Taste evaluation of the processed plant-protein-containing liquid composition obtained in the above (1) was performed. The evaluation was performed by four panelists, and comparison of whether or not there was a change in taste of the processed plant-protein-containing liquid composition of each of Examples and Comparative Examples (specifically, whether or not the bitterness became stronger) was performed based on the taste (specifically, bitterness) of the processed plant-protein-containing liquid composition obtained by using the protein deamidase alone without using a protease. Specifically, a case where there was a change in taste was evaluated as "×", and a case where there was no change in taste was evaluated as "○". The results are shown in Tables 2A to 4A.

### (4) Emulsifiability evaluation

In a 400 mL beaker, 300 mL of the processed plant-protein-containing liquid composition obtained in the above (1) was put, and the beaker was set in a homogenizer manufactured by SILVERSON. The homogenizer timer was set to 5 minutes, the stirring number was set to 5,000 RPM, and the processed plant-protein-containing liquid composition was homogenized. After homogenization, the beaker containing the processed plant-protein-containing liquid composition was taken out, a part of the liquid composition was taken out for emulsifiability evaluation, and the absorbance at a wavelength of 600 nm was measured. The results are shown in Tables 2B to 4B.

### (5) Evaluation of improvement in dispersion stability

The supernatant of the sample obtained by refrigerating the processed plant-protein-containing liquid composition prepared in the above (4) for 1 week was collected. Turbidity was measured using Portable Microprocessor Turbidity Meter (manufactured by Hanna Instruments, Inc.). The results are shown in Tables 2B to 4B.

### (6) Evaluation of improvement in foamability

The processed plant-protein-containing liquid composition obtained in the above (1) was sufficiently stirred until the liquid composition was homogeneous. After stirring, 70 mL of the liquid composition was measured using a measuring cylinder, and poured into Milk Frother manufactured by HadinEEon, and foaming was performed for 1 minute. After foaming, the processed plant-protein-containing liquid composition was poured into a measuring cylinder, and the volume of foam was measured. The results are shown in Tables 2B to 4B.

**[Table 2A]**

| | | Comparative Example 1 | Comparative Example 2 | **Example 1** | **Example 2** | **Example 3** | **Example 4** | **Example 5** | **Example 6** | **Example 7** |
|---|---|---|---|---|---|---|---|---|---|---|
| Pea protein material (wt%) | | 3.13 | 3.13 | **3.13** | **3.13** | **3.13** | **3.13** | **3.13** | **3.13** | **3.13** |
| Pea protein (wt%) | | 2.5 | 2.5 | **2.5** | **2.5** | **2.5** | **2.5** | **2.5** | **2.5** | **2.5** |
| Protein deamidase (U/g protein) | | - | 5.0 | **5.0** | **5.0** | **5.0** | **5.0** | **5.0** | **5.0** | **5.0** |
| Protein deamidase (U/g protein material) | | - | 4.0 | 4.0 | 4.0 | 4.0 | 4.0 | 4.0 | 4.0 | 4.0 |
| Protease 1 (U/g protein) | | - | - | **15** | **50** | **100** | - | - | - | - |
| Protease 1 (U/g protein material) | | - | - | **12.0** | **39.9** | **79.9** | - | - | - | - |
| Protease 2 (U/g protein) | | - | - | - | - | - | **15** | **50** | **100** | **200** |
| Protease 2 (U/g protein material) | | - | - | - | - | - | **12.0** | **39.9** | **79.9** | **159.7** |
| Results | Soluble protein amount | 0.95% | 1.23% | **1.48%** | **1.70%** | no data | **1.72%** | **1.84%** | **1.95%** | **1.91%** |
| | Relative value of soluble protein amount | | 1 | **1.2** | **1.38** | no data | **1.4** | **1.5** | **1.59** | **1.55** |
| | Taste change suppressing effect | | Reference | ○ | ○ | ○ | ○ | ○ | ○ | ○ |

**[Table 2B]**

| | | Comparative Example 1 | Comparative Example 2 | **Example 5** |
|---|---|---|---|---|
| | Emulsifiability (OD 600 nm) | 2.91 | 2.95 | **2.96** |
| | Dispersion stability | 195 | 291 | **26,100** |
| Results | Dispersion stability improvement index | | 1 | **89.7** |
| | Foamability (mL) | 5 | 50 | **70** |
| | Foamability improvement index | | 1 | **1.4** |

**[Table 3A]**

| | | Comparative Example 3 | Comparative Example 4 | **Example 8** | **Example 9** | Comparative Example 5 | **Example 10** | **Example 11** | **Example 12** |
|---|---|---|---|---|---|---|---|---|---|
| Soybean protein material (wt%) | | 3.13 | 3.13 | **3.13** | **3.13** | 3.13 | **3.13** | **3.13** | **3.13** |
| Soybean protein (wt%) | | 1.21 | 1.21 | **1.21** | **1.21** | 1.21 | **1.21** | **1.21** | **1.21** |
| Protein deamidase (U/g protein) | | - | 10.4 | **10.4** | **10.4** | - | **10.4** | **10.4** | **10.4** |
| Protein deamidase (U/g protein material) | | - | 4.0 | 4.0 | 4.0 | - | 4.0 | 4.0 | 4.0 |
| Protease 1 (U/g protein) | | - | - | **31.1** | **104** | 207 | **207** | - | - |
| Protease 1 (U/g protein material) | | - | - | **12.0** | **40.2** | 80.0 | **80.0** | - | |
| Protease 2 (U/g protein) | | - | - | - | | - | - | **31.1** | **104** |
| Protease 2 (U/g protein material) | | - | - | - | | - | - | **12.0** | **40.2** |
| Results | Soluble protein amount | 0.71% | 0.77% | **1.02%** | **1.41%** | 0.93% | **1.48%** | **1.82%** | **1.72%** |
| | Relative value of soluble protein amount | | 1 | **1.32** | **1.83** | 1.21 | **1.92** | **2.36** | **2.23** |
| | Taste change suppressing effect | | Reference | ○ | ○ | × | ○ | ○ | ○ |

**[Table 3B]**

| | | Comparative Example 3 | Comparative Example 4 | **Example 12** |
|---|---|---|---|---|
| | Emulsifiability (OD 600 nm) | 2.65 | 2.77 | **2.85** |
| | Dispersion stability | 15 | 25 | **476** |
| Results | Dispersion stability improvementindex | | 1 | **19** |
| | Foamability (mL) | 150 | 210 | **230** |
| | Foamability improvement index | | 1 | **1.09** |

**[Table 4A]**

| | | Comparative Example 6 | Comparative Example 7 | **Example 13** | **Example 14** | **Example 15** | **Example 16** | **Example 17** |
|---|---|---|---|---|---|---|---|---|
| Almond protein material (wt%) | | 6.11 | 6.11 | **6.11** | **6.11** | **6.11** | **6.11** | **6.11** |
| Almond protein (wt%) | | 2.5 | 2.5 | **2.5** | **2.5** | **2.5** | **2.5** | **2.5** |
| Protein deamidase (U/g protein) | | | 5.0 | **5.0** | **5.0** | **5.0** | **5.0** | **5.0** |
| Protein deamidase (U/g protein material) | | - | 2.0 | **2.0** | **2.0** | **2.0** | **2.0** | **2.0** |
| Protease 1 (U/g protein) | | - | - | **15** | **50** | **100** | - | - |
| Protease 1 (U/g protein material) | | - | - | **6.1** | **20.5** | **40.9** | - | |
| Protease 2 (U/g protein) | | - | - | - | **-** | **-** | **15** | **50** |
| Protease 2 (U/g protein material) | | - | - | - | **-** | **-** | **6.1** | **20.5** |
| Results | Soluble protein amount | 1.33% | 2.61% | **2.66%** | **2.65%** | **2.68%** | **2.90%** | **2.92%** |
| | Relative value of soluble protein amount | | 1 | **1.02** | **1.02** | **1.03** | **1.11** | **1.12** |
| | Taste change suppressing effect | | Reference | ○ | ○ | ○ | ○ | ○ |

**[Table 4B]**

| | | Comparative Example 6 | Comparative Example 7 | **Example 17** |
|---|---|---|---|---|
| | Emulsifiability (OD 600 nm) | 2.77 | 2.76 | **2.77** |
| | Dispersion stability | 67 | 3640 | **5120** |
| Results | Dispersion stability improvementindex | | 1 | **1.41** |
| | Foamability (mL) | 10 | 18 | **30** |
| | Foamability improvement index | | 1 | **1.67** |

As is clear from Tables 2A, 3A and 4A, by treating the pea protein-containing liquid composition, the soybean protein-containing liquid composition, and the almond protein-containing liquid composition with the protein deamidase and the protease derived from Bacillus licheniformis (protease 1) or the protease derived from Bacillus amyloliquefaciens (protease 2), as compared with the case of using the protein deamidase alone, it was possible to suppress the change in taste while enhancing the solubility of the protein (Examples 1 to 17). As is clear from Tables 2B, 3B, and 4B, by treating the pea protein-containing liquid composition, the soybean protein-containing liquid composition, and the almond protein-containing liquid composition with the protein deamidase and the protease derived from Bacillus amyloliquefaciens (protease 2), the emulsifiability itself was almost not changed, but improvement in dispersion stability and foamability was recognized (Examples 5, 12, and 17).

## Claims

1. A taste change suppressing method of suppressing a change in taste while improving solubility of a plant-protein-containing liquid composition treated with a protein deamidase, the taste change suppressing method comprising:
a step of treating the plant-protein-containing liquid composition with the protein deamidase and a bacteria-derived protease.

2. The taste change suppressing method according to claim 1, wherein the bacteria-derived protease is selected from the group consisting of proteases derived from Bacillus licheniformis, Bacillus amyloliquefaciens, and corresponding genus Geobacillus.

3. The taste change suppressing method according to claim 1, wherein the plant protein is selected from the group consisting of pea protein, soybean protein, and almond protein.

4. The taste change suppressing method according to claim 1, wherein a used amount of the bacteria-derived protease per 1 g of the plant protein is 13 to 250 U.

5. The taste change suppressing method according to claim 1, wherein a used amount of the protein deamidase per 1 g of the plant protein is 4 U or more.

6. The taste change suppressing method according to claim 1, wherein a used amount of the bacteria-derived protease per 1 U of the protein deamidase is 1 to 45 U.

7. A taste change inhibitor comprising a bacteria-derived protease and used for suppressing a change in taste while improving solubility of a plant-protein-containing liquid composition treated with a protein deamidase.

8. The taste change inhibitor according to claim 7, wherein the bacteria-derived protease is selected from the group consisting of proteases derived from Bacillus licheniformis, Bacillus amyloliquefaciens, and corresponding genus Geobacillus.

9. The taste change inhibitor according to claim 7, wherein the plant protein is selected from the group consisting of pea protein, soybean protein, and almond protein.

10. The taste change inhibitor according to claim 7, which is further used for improving foamability of a plant-protein-containing liquid composition treated with a protein deamidase.

11. The taste change inhibitor according to claim 7, which is further used for improving dispersion stability of a plant-protein-containing liquid composition treated with a protein deamidase.
